# EUROPEAN PATENT APPLICATION

(11) **EP 4 316 574 A1**
(43) Date of publication of application: **07.02.2024**
(21) Application number: 22781222.9
(22) Date of filing: 31.03.2022
(51) Int. Cl.: A61N 1/39, A61B 5/01, A61B 18/14

(54) **IN-VIVO TEMPERATURE CONTROL SYSTEM**

(30) Priority: 31.03.2021 JP 2021059751
(71) Applicant: Toray Industries, Inc., Tokyo 103-8666 (JP)
(72) Inventor: SAKAKIBARA, Hajime, Otsu-shi, Shiga 520-2141 (JP); NOMURA, Tatsufumi, Tokyo 103-8666 (JP); NAKAJIMA, Hiroki, Otsu-shi, Shiga 520-2141 (JP); MATSUKUMA, Akinori, Otsu-shi, Shiga 520-2141 (JP); MIKAMI, Jun, Otsu-shi, Shiga 520-2141 (JP)
(74) Representative: Kador & Partner Part mbB
(86) International application number: PCT/JP2022/016468
(87) International publication number: WO 2022/211012

(57) **Abstract**

The present invention provides an *in vivo* temperature control system that appropriately controls the internal esophageal temperature and has an aspiration prevention function. The *in vivo* temperature control system of the present invention includes: a catheter insertable into a living body; a temperature sensor that can measure the temperature in the living body; a liquid storage section for storing a liquid; a pump for supplying the liquid from the liquid storage section to the catheter; and a control section for controlling the driving of the pump; in which the control section calculates the difference obtained by subtracting the integrated amount of a liquid sucked to the inside of the catheter from the integrated amount of a liquid released to the outside of the catheter, and allows the pump to operate and suck liquid into the catheter when the difference exceeds a preset threshold, and then stop the driving of the pump when the difference reaches the preset threshold or less.

## Description

### TECHNICAL FIELD

The present invention relates to an *in vivo* temperature control system capable of determining suction of a liquid based on differential information on the amount of the liquid.

### BACKGROUND ART

Atrial fibrillation is a kind of arrhythmia, and known to involve repeated irregular contraction of the atrium, leading to poor blood circulation, hence causing discomfort or malaise. Therefore, methods of treating atrial fibrillation have been widely carried out by catheter ablation procedures, wherein the pulmonary vein and the cardiac muscle tissues in the vicinity thereof such as the posterior wall of the left atrium, which are major sources of occurrence of atrial fibrillation, are ablated (pulmonary vein isolation).

On the other hand, since the site of ablation (left atrium) and the esophagus are positioned close to each other during the treatment by a catheter ablation procedure, it has been pointed out that there is a risk of injury of the esophagus, leading to severe esophageal complications such as left atrial-esophageal fistula and esophagus vagus nerve paralysis. Thus, appropriate control of the temperature in the esophagus is required.

Examples of means for the control of the temperature in the esophagus that have been reported include a temperature measurement device that employs an approach through the nose or mouth of the patient (nasal or oral approach) to insert a catheter equipped with a temperature sensor into the esophagus. The temperature sensor measures the internal esophageal temperature, and an alert is output to the outside when the internal temperature is judged to have reached a threshold (Patent Document 1).

A device that calculates the remaining time until the internal esophageal temperature reaches the temperature limit based on the rate of change of the internal esophageal temperature and outputs it to the outside has also been reported (Patent Document 2).

### PRIOR ART REFERENCES

### PATENT DOCUMENTS

[Patent Document 1] JP 6618254 B
[Patent Document 2] JP 6804910 B

### SUMMARY OF THE INVENTION

### PROBLEMS TO BE SOLVED BY THE INVENTION

In order to appropriately control the internal esophageal temperature during the treatment by a catheter ablation procedure, it is required that the internal esophageal temperature be continuously monitored, and proactive measures be taken before the intraesophageal tissue are damaged.

The temperature measurement device described in Patent Document 1 is capable of monitoring the internal esophageal temperature during the ablation therapy and outputting the alert to the outside when the internal temperature is judged to have reached a threshold, to thereby allow one to take prior measures such as stopping of the ablation before the esophagus is injured by heating or cooling. However, the esophagus temperature may be rapidly changed during ablation therapy, and thus a delay in noticing of the alert may lead to a delay in the required measures.

The device described in Patent Document 2 can calculate the time until the temperature reaches a dangerous temperature at which the esophagus is damaged based on the rate of change of the internal esophageal temperature and output it to the outside, to thereby allow for prediction of rapid changes of the esophagus temperature and time to take the required measures. However, since the device does not have a mechanism for controlling the internal esophageal temperature within safe temperatures, the risk of esophagus damage cannot be reduced in case where the measures are delayed.

Accordingly, an object of the present invention is to provide an *in vivo* temperature control system that has means for monitoring the internal temperature of a living body such as the esophagus, and for directly controlling the temperature in the organ side by injecting a liquid, and that calculates the difference obtained by subtracting the integrated amount of a liquid sucked to the inside of the catheter from the integrated amount of liquid released outside the catheter, and allows the pump to operate and suck a liquid into the catheter when the difference exceeds a preset threshold, and then stop the driving of the pump when the difference reaches the preset threshold or less.

### MEANS FOR SOLVING THE PROBLEMS

In order to solve the above problem, the present inventors intensively studied to discover the following inventions (1) to (4):
(1) An *in vivo* temperature control system, comprising:
   a catheter insertable into a living body;
   a temperature sensor that can measure the temperature in the living body;
   a liquid storage section for storing a liquid;
   a pump for supplying the liquid from the liquid storage section to the catheter; and
   a control section for controlling the driving of the pump;
   wherein the control section calculates the difference obtained by subtracting the integrated amount of a liquid sucked to the inside of the catheter from the integrated amount of liquid released outside the catheter, and allows the pump to operate and suck liquid into the catheter when the difference exceeds a preset threshold, and then stop the driving of the pump when the difference reaches the preset threshold or less.
(2) The *in vivo* temperature control system of (1), comprising a pressure sensor for detecting the internal pressure of the catheter,
   wherein the control section when sucking liquid into the catheter sets the integrated amount of liquid released outside the catheter and the integrated amount of a liquid sucked to the inside of the catheter to 0 when the pressure value obtained by the pressure sensor reaches a set threshold or less.
(3) The *in vivo* temperature control system of (1), comprising a flow velocity meter or a flow rate meter that can measure the flow rate of the liquid flowing inside the catheter;
   wherein the control section when sucking liquid from the outside of the catheter sets the integrated amount of liquid released outside the catheter and the integrated amount of a liquid sucked to the inside of the catheter to 0 when the flow rate or flow rate value obtained by the flow velocity meter or the flow rate meter reaches a preset threshold or less.
(4) The *in vivo* temperature control system of any of (1) to (3), wherein the control section selects whether the liquid in the liquid storage section is released or the liquid is sucked and drives the pump based on a signal detected by the temperature sensor or preset time.

### EFFECT OF THE INVENTION

According to the present invention, a temperature sensor can measure the internal temperature in a living body, and liquid suction when adjusting to a predetermined temperature using a liquid can be automatically determined.

### BRIEF DESCRIPTION OF THE DRAWINGS

Fig. 1 is a diagram illustrating the external appearance of an *in vivo* temperature control system according to a first embodiment of the present invention.
Fig. 2 is a schematic diagram illustrating the internal structure of the *in vivo* temperature control system illustrated in Fig. 1.
Fig. 3 is a flow chart illustrating an operational procedure of the control section in the first control operation of the *in vivo* temperature control system of the present invention.

### MODE FOR CARRYING OUT THE INVENTION

Specific embodiments of the present invention are described below with reference to the drawings. However, the present invention is not limited to these modes. The present invention may be modified as appropriate without departing from the scope in which the effect of the invention can be produced. It should be noted that the same reference numerals are used for the same elements.

### <First Embodiment>

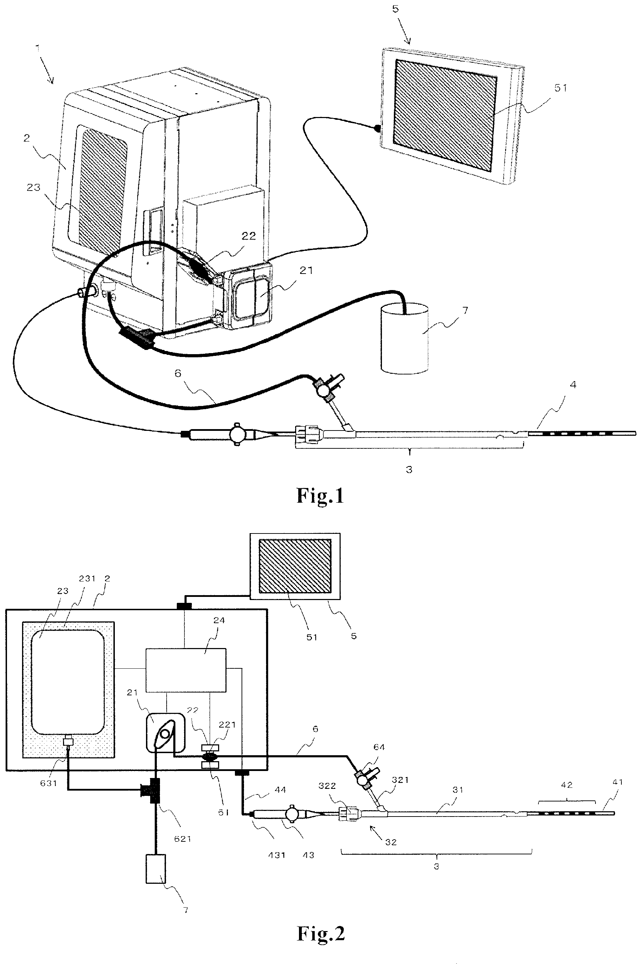

Fig. 1 is a diagram illustrating the external appearance of an *in vivo* temperature control system 1 according to a first embodiment of the present invention. For example, when an ablation procedure is carried out using a balloon ablation catheter in which the inside of the balloon is heated using a radiofrequency, the *in vivo* temperature control system 1 may be used for monitoring the internal esophageal temperature in a position close to the heart to be treated by the ablation, and also for cooling the internal esophageal temperature with a liquid. The biological organ to which the *in vivo* temperature control system 1 is applicable is not limited. The system may be applied to the pharynx, larynx, lung, esophagus, stomach, or the like. It is especially preferably used for cooling of the inside of the esophagus.

The *in vivo* temperature control system 1 herein comprises: an *in vivo* temperature control device 2 for sending or sucking a temperature-controlled liquid to a catheter 3; a catheter 3 on which a pore(s) through which a liquid can be sent into or sucked from the outside is/are formed, the catheter being insertable into the living body; a temperature probe 4 containing a temperature sensor 42, the probe being insertable into the catheter 3; a monitor 5 capable of displaying a signal from the temperature probe 4; a liquid-sending-sucking dual-purpose tube 6 connected to a pump 21 and a pressure center 22, the tube connecting the *in vivo* temperature control device 2 to the catheter 3; and a waste liquid section 7.

Fig. 2 is a schematic diagram illustrating the internal structure of the *in vivo* temperature control device 2 illustrated in Fig. 1.

The *in vivo* temperature control device 2 comprises: a pump 21 for sending or sucking a liquid to the catheter 3; a pressure sensor 22 for detecting the internal pressure of the catheter 3; a liquid storage section 23 for storing the temperature-controlled liquid; and a control section 24 for controlling the driving of the pump.

The pump 21 contained in the *in vivo* temperature control device 2 is a roller-type tube pump. By the forward rotation of the roller of the pump 21, a liquid can be sent from the liquid storage section 23 to the catheter 3. By the reverse rotation of the roller of the pump 21, a liquid can be sucked from the distal end portion or the pore(s) of the catheter 3.

In an embodiment other than the first embodiment, the *in vivo* temperature control device may have a liquid-sending catheter having a pore(s) through which a liquid can be sent to the outside and a liquid-sucking catheter having a pore(s) through which a liquid can be sucked from the outside as catheters, and a liquid-sending pump for sending a liquid from the liquid storage section 23 to the liquid-sending catheter and a liquid-sucking pump for sucking a liquid through the distal end portion or the pore(s) of the liquid-sucking catheter, as pumps. In this case, the liquid-sending pump can be driven to release a liquid from the liquid storage section to the outside of the liquid-sending catheter, and the liquid-sucking pump can be driven to suck a liquid released to the outside into the liquid-sucking catheter. This allows for efficient temperature control in the living body.

The pressure sensor 22 included in the *in vivo* temperature control device 2 comprises a contact-type displacement gauge 221, and a bulge section 61 of the liquid-sending-sucking dual-purpose tube 6 in contact with the contact-type displacement gauge 221. The bulge section 61 of the liquid-sending-sucking dual-purpose tube 6 is a portion prepared by forming a part of the liquid-sending-sucking dual-purpose tube 6 into a bag shape. The bulge section 61 is designed such that it expands or shrinks in accordance with the pressure in the tube. Thus, by detecting the amount of displacement of the bulge section 61 as a signal by the contact-type displacement gauge 221, the internal pressure of the catheter 3 connected to the liquid-sending-sucking dual-purpose tube 6 can be measured through the liquid-sending-sucking dual-purpose tube 6.

The detection method by the pressure sensor 22 is not limited. Any detection method may be employed as long as the internal pressure of the catheter 3 during the liquid sending or sucking operation can be detected and sent to the control section 24 as a signal. For example, as an embodiment other than the first embodiment, a method in which a diaphragm-type inline pressure sensor is added, or a method in which a thin tube is branched from the liquid-sending-sucking dual-purpose tube 6, and the pressure in the thin tube is measured, may be employed.

In an embodiment other than the first embodiment, the *in vivo* temperature control device 2 may comprise a flow rate meter that can measure the flow rate of the liquid flowing inside the catheter 3, instead of the pressure sensor 22. As the flow rate meter, for example, an ultrasonic flow rate meter can be used, which is connected to the catheter 3 or the liquid-sending-sucking dual-purpose tube 6 and can detect the difference in the transmission time of ultrasonic signals to thereby measure the flow rate of a liquid flowing inside the catheter 3.

The detection method with a flow rate meter is not limited. Any detection method may be employed as long as the flow rate through the catheter 3 during the liquid sending or sucking operation can be detected and sent to the control section 24 as a signal. A flow velocity meter that can detect the velocity of the flow through the catheter 3 may be provided, which velocity may be sent to the control section 24 as a signal.

The liquid storage section 23 of the *in vivo* temperature control system 1 of the first embodiment is a general, commercially available infusion bag for physiological saline, glucose solution, or the like. The liquid storage section 23 has a structure by which it can be housed inside the *in vivo* temperature control device 2. In the *in vivo* temperature control device 2, a Peltier device 231 and an in-device temperature sensor, not shown, for measurement of the temperature in the liquid storage section 23 are placed such that they are in contact with the liquid storage section 23. By controlling the temperature of the Peltier device 23 1 based on a signal detected from the in-device temperature sensor, temperature control of the liquid stored in the liquid storage section 23 can be carried out. In cases where a hot ablation procedure is carried out, the temperature of the liquid in the liquid storage section 23 is controlled preferably at 0°C to 15°C, more preferably at 0°C to 10°C.

The liquid storage section 23 may be in any shape as long as it is capable of storing a liquid such as physiological saline. The liquid storage section 23 may be housed inside the *in vivo* temperature control device 2, or may be placed outside the *in vivo* temperature control device 2. In cases where the liquid storage section 23 is housed inside the *in vivo* temperature control device 2, it is preferred that temperature control of the liquid in the liquid storage section 23 can be made as described above. In an embodiment other than the first embodiment, the infusion bag may be cooled using a coolant such as ice instead of the Peltier device 231, or an infusion bag that has been preliminarily frozen may be used while thawing it. The liquid used may be purified water or tap water instead of the physiological saline or glucose solution.

In cases of use in cryoablation that requires the inside of the living body to be warmed to a higher level than usual, the *in vivo* temperature control system of the present invention may be used therefor. In such cases, the liquid stored in the liquid storage section 23 may be heated to a temperature of 30°C to 45°C using a heating resistor.

The control section 24 included in the *in vivo* temperature control device 2 controls the pump 21, and have a circuit for measuring the volume of the liquid released to the outside of the catheter 3, that is, the volume of the liquid released to the inside of the living body (injection volume), and the volume of the liquid sucked to inside of the catheter 3, that is, the volume of the liquid sucked from the inside of the living body (suction volume) based on the driving history of the pump (for example, rotation speed and the driving time of the pump), and for saving the integrated amount of liquid. The control section also has a circuit for controlling the driving of the pump based on the information on the difference (Δ) obtained by subtracting the integrated amount of the suction volume from the integrated amount of the injection volume.

Further, the control section 24 preferably comprises a circuit for controlling the driving of the pump 21 based on a signal detected by the temperature probe 4 or the passage of time. By this, for example, the control section 24 can select whether the liquid in the liquid storage section 23 is released or sucked, and control driving of the pump 21 based on a signal detected by the temperature probe 4 or the passage of time, allowing for automatic control of whether the liquid in the outside is sucked through the catheter 3 or the liquid is released to the outside. Further, the control section 24 preferably comprises a circuit for controlling the driving of the pump 21 based on a signal detected by the pressure sensor 22. By this, for example, the control section 24 of the *in vivo* temperature control system 1 according to the first embodiment can have a mechanism that allows numerical conversion of the information from the liquid-sending-sucking dual-purpose tube 6 on the amount of displacement to pressure information. In the first embodiment, it can be estimated whether or not a liquid remains in the living body based on the pressure information when a liquid is sucked into the catheter 3. By this, for example, in a case where a negative pressure is detected during suction of a liquid, the control section 24 can judge that there is no more liquid that can be sucked from the inside of the living body, and stop driving of the pump 21. Specifically, the control section 24 can have preset suction stop pressure. In cases where the internal tube pressure is judged to be not more than the preset threshold of the suction stop pressure, the control section 24 stops the driving of the pump 21. In this case, the control section 24 preferably calculates the difference (Δ) to be 0 by judging that the integrated amount of injection volume and the integrated amount of suction volume are 0.

The catheter 3 is a cylindrical member insertable into a living body by a nasal or oral approach, and capable of sending or sucking a liquid through the distal end of the catheter or a pore(s) formed on the surface, through a lumen. More specifically, the catheter 3 has a structure comprising a tube section 31 insertable into a living body, and a valved connector 32 fixed at the proximal end side in the longitudinal direction of the tube section 31.

The material used for the tube section 31 is not limited as long as it is a flexible material nasally or orally insertable into a living body, and examples of the material include thermoplastic resins such as polyvinyl chloride, polyurethane, and silicone. For the confirmation of the site of placement in the living body, the material preferably contains a radiopaque material.

For example, in cases where the tube section 31 is nasally inserted into the living body from the nose, the length of the tube section 31 is preferably about 200 mm to 1000 mm; the outer diameter is preferably about 1.7 mm to 6.0 mm; and the inner diameter is preferably about 1.0 mm to 5.0 mm.

The valved connector 32 is fixed at the proximal end side of the tube section 31, and connectable to the liquid-sending-sucking dual-purpose tube 6. The valved connector 32 comprises a port 321 for sending or sucking of the liquid from the distal end side of the tube section 31, and a valve 322 for fixing the temperature probe 4 in the case where the temperature probe 4 is inserted to the catheter 3. The valve 322 is preferably openable and closable by a rotational motion or the like. The above configuration enables manipulation of the temperature probe 4 when the valve 322 is open, and enables fixation of the temperature probe 4 when the valve 322 is closed.

In an embodiment other than the first embodiment, the *in vivo* temperature control device may comprise, as catheters, a liquid-sending catheter for releasing a liquid to the outside, a liquid-sucking catheter for sucking a liquid from the outside, a first port for sending a liquid from the distal end side of the liquid-sending catheter, and a second port for sucking a liquid from the distal end side of the liquid-sucking catheter. In such a case where a liquid-sending catheter and a liquid-sucking catheter are separately included instead of the liquid-sending-sucking dual-purpose tube 6, a liquid-sending tube connected to the liquid storage section 23 and the liquid-sending pump is connected to the first port, and a liquid-sucking tube connected to the liquid-sucking pump and the waste liquid section 7 is connected to the second port, whereby the pathway for sending a liquid and the pathway for sucking a liquid can be independently operated, allowing for sending of a cooled liquid into the living body at all times.

The temperature probe 4 is a member to be nasally or orally inserted into a living body to measure the internal temperature inside the living body. The temperature probe comprises a shaft section 41 to be inserted into the living body, a temperature sensor 42 placed at the distal end side, and a handle section 43.

The material used for the shaft section 41 is not limited as long as it is a flexible material nasally or orally insertable into a living body, and examples of the material include thermoplastic resins such as polyether block amide, polyurethane, nylon, polyolefin, polyamide, and polyetheramide.

The outer diameter of the shaft section 41 is preferably about 1.0 mm to 4.0 mm, more preferably a diameter that allows insertion of the shaft section 41 into a lumen of the catheter 3. The shaft section 41 preferably has a length of about 300 mm to 1100 mm. In cases where it is inserted into a lumen of the catheter 3, the temperature sensor 42 on the shaft section 41 is preferably placed at a position where it protrudes from the distal end side of the catheter 3.

The shaft section 41 may have a function by which the distal end side can be deflected by operation of the handle section 43. By this, in particular, in cases of application to the esophagus, the risk of straying into the airway can be reduced when the temperature probe 4 is nasally or orally inserted into the esophagus. Moreover, the esophagus is meandering, rather than being straight, between the pharynx and the gastric cardia. By the deflection operation, placement of the temperature sensor 42 at a desired esophageal site is possible.

One or more temperature sensors 42 are attached to the distal end side of the shaft section 41. In order to allow measurement of the internal temperature in a larger area inside a living body, a plurality of temperature sensors 42 are preferably contained.

The material used for the temperature sensor 42 is not limited as long as it has good thermal conductivity. It is preferably a radiopaque material from the viewpoint of measurement of the temperature at a position close to the ablation site.

The handle section 43 comprises a connector 431 for connection to the *in vivo* temperature control device 2. In the *in vivo* temperature control system 1 according to the first embodiment, the *in vivo* temperature control device 2 is connected to the temperature probe 4 through a connection cable 44.

In the first embodiment, the catheter 3 for releasing a liquid to the outside and the temperature probe 4 in which the temperature sensors 42 are placed are independent, and are configured such that the temperature probe 4 is inserted into the catheter 3. In another embodiment, temperature sensors may be placed on the tube section of the catheter 3. In this case, a pore(s) through which a liquid can be sent into a living body is/are preferably placed at the proximal end side in the longitudinal direction of each temperature sensors placed on the catheter 3.

The monitor 5 is capable of displaying information on the internal temperature in the living body detected by the temperature probe 4, as a digital number, bar graph, or trend graph. The monitor 5 has a function by which, when the temperature in the living body has exceeded a preset threshold, the display color is changed to present the temperature change to an operator as visual information. For example, the monitor may be set such that the color changes from a cold color to a warm color as the temperature increases from a low temperature to a high temperature. By this, the temperatures detected by the temperature probes 4 placed along the longitudinal direction in the living body can be known as the temperatures at the corresponding positions in the living body. Therefore, the operator can perceive which site of the living body has a higher temperature than the others. Further, since the temperature is presented not only as a digital number, but also as a bar graph, the temperature can be compared among different sites in the living body. Further, the operator can be visually informed of the fact that the internal temperature of the living body has increased to a level at which the risk of injury of the biological organ is high.

The monitor 5 preferably has a function for presenting, to the operator, information on the operation of sending and sucking of the liquid; information on errors that have occurred in the system, and alerts including alarms; and operational information such as the operation time, the number of times of sending and sucking of the liquid, and the amounts of liquid sent and sucked. By this, the operational conditions, malfunctioning conditions, and dangerous conditions can be visually or aurally informed to the operator.

The monitor 5 preferably comprises a touch-screen display 51 with which various parameters related to the system operation can be input, and with which the input parameters can be transmitted to the control section 24 of the *in vivo* temperature control device 2. This enables the operator to start and stop the operation, and to set and modify various parameters, of the *in vivo* temperature control device 2 from a distant location.

The liquid-sending-sucking dual-purpose tube 6 is a tube for delivering a liquid from the liquid storage section 23 to the catheter 3 through the pump 21 during the sending of the liquid, and delivering a liquid from the catheter 3 to the waste liquid section 7 during the suction of the liquid. The waste liquid section 7 is a site for storing the unnecessary liquid after the suction of the liquid from the body.

The liquid-sending-sucking dual-purpose tube 6 contained in the *in vivo* temperature control system 1 comprises a bulge section 61, a channel switching section 62, a liquid supply port 63 for connection to the liquid storage section 23, and a connection port 64 for connection to the catheter 3.

As described above, the bulge section 61 is a tube formed into a bag shape, and designed such that it expands or shrinks in accordance with the pressure in the tube. Thus, by detecting the amount of displacement of the bulge section 61 by the contact-type displacement gauge 221, the internal pressure of the catheter 3 connected to the liquid-sending-sucking dual-purpose tube 6 can be measured through the liquid-sending-sucking dual-purpose tube 6.

The channel switching section 62 in the first embodiment is a three-way check valve 621, and connected to the primary side of the pump 21. Therefore, when the liquid is to be sent, forward rotation of the pump 21 switches the channel of the three-way check valve 621 to the direction in which the liquid storage section 23 is connected to the pump 21, allowing the liquid to flow to the outside of the catheter 3. Further, when the liquid is to be sucked to the inside of the catheter 3, reverse rotation of the pump 21 switches the channel of the three-way check valve 621 to the direction in which the pump 21 is connected to the waste liquid section 7, allowing the sucked liquid to be discharged to the waste liquid section 7.

The liquid supply port 63 may be in any form as long as the liquid can be supplied from the liquid storage section 23 into the liquid-sending-sucking dual-purpose tube 6. In the first embodiment, since the liquid storage section 23 is an infusion bag, the liquid supply port 63 is preferably a needle 631 capable of piercing the infusion bag.

The connection port 64 may be in any form as long as it can be connected to the catheter 3. It is preferably a three-way stopcock. In this case, when malfunction of the *in vivo* temperature control system occurs, a syringe or the like can be connected so as to enable manual sending and sucking of the liquid.

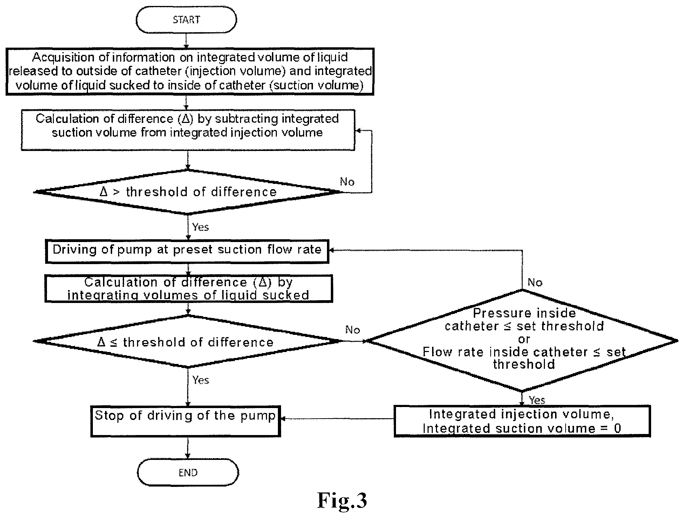

A flow chart illustrating an example of the operational procedure for the control section 24 that determines suction of a liquid based on differential information between the volumes of a liquid to the outside (injected liquid) and of a liquid sucked from the outside (sucked liquid) is described below using Fig. 3.

In this example of the operational procedure, the control section 24 obtains the integrated amount of a liquid released to the outside of the catheter 3 (injection volume) and the integrated amount of a liquid sucked to the inside of the catheter 3 (suction volume). The control section 24 has functions to calculate the integrated injection volume and the integrated suction volume based on the driving history of the pump 21 (for example, the rotation speed and the driving time of the pump) and save them. The control section 24 judges whether or not the difference (Δ) obtained by subtracting the obtained integrated suction volume from the obtained integrated injection volume reaches a preset threshold of the difference, in other words, whether or not the condition for the start of sucking a liquid is satisfied, by comparison of the values. In cases where the control section 24 judges that the difference exceeds the preset threshold of the difference, the control section 24 drives the pump 21 to start suction of a liquid from the inside of the living body at a preset suction flow rate.

After the start of suction, the control section 24 calculates the suction volume based on the driving history of the pump 21, and calculates the difference (Δ). In cases where the difference (Δ) reaches the preset threshold or less, the control section 24 stops the driving of the pump 21, in other words, stops suction from the inside of the living body. On the other hand, in cases where the difference (Δ) does not reach the preset threshold or less, the control section 24 calculates the internal pressure of the catheter 3, and judges whether or not the internal pressure of the catheter 3 is not more than the preset threshold of the suction stop pressure, in other words, whether or not the second condition for stopping suction is satisfied. In cases where the internal pressure of the catheter 3 is judged to be not more than the preset threshold of the suction stop pressure, the control section 24 sets the integrated injection volume and the integrated suction volume to 0, and stops the driving of the pump 21.

In embodiments where the *in vivo* temperature control device 2 comprises a flow rate meter that can measure the flow rate through the catheter 3, whether or not the flow rate through the catheter 3 is not more than the preset threshold of the suction stop flow rate is judged. In cases where the flow rate through the catheter 3 is judged to be not more than the preset threshold of the suction stop flow velocity, the control section 24 sets the integrated injection volume and the integrated suction volume to 0, and stops the driving of the pump 21.

In another embodiment where a liquid-sending pump and a liquid-sucking pump are included as pumps and a liquid-sending catheter and a liquid-sucking catheter are included as catheters, the control section 24 obtains the integrated amount of a liquid released to the outside of the liquid-sending catheter (injection volume) and the integrated amount of a liquid sucked to the inside of the liquid-sucking catheter (suction volume). The control section 24 judges whether or not the difference (Δ) obtained by subtracting the obtained integrated suction volume from the obtained integrated injection volume reaches a preset threshold of the difference, in other words, whether or not the condition for the start of sucking a liquid is satisfied, by comparison of the values. In cases where the control section 24 judges that the difference exceeds the preset threshold of the difference, the control section 24 drives the liquid-sucking pump to start suction of a liquid from the inside of the living body at a preset suction flow rate.

After the start of suction, the control section 24 calculates the suction volume based on the driving history of the liquid-sucking pump, and calculates the difference (Δ). In cases where the difference (Δ) reaches the preset threshold or less, the control section 24 stops the driving of the liquid-sucking pump, in other words, stops suction from the inside of the living body. On the other hand, in cases where the difference (Δ) does not reach the preset threshold or less, the control section 24 calculates the internal pressure of the liquid-sucking catheter, and judges whether or not the internal pressure of the liquid-sucking catheter is not more than the preset threshold of the suction stop pressure, in other words, whether or not the second condition for stopping suction is satisfied. In cases where the internal pressure of the liquid-sucking catheter is judged to be not more than the preset threshold of the suction stop pressure, the control section 24 sets the integrated injection volume and the integrated suction volume to 0, and stops the driving of the liquid-sucking pump.

In still another embodiment where the *in vivo* temperature control device 2 comprises a flow rate meter that can measure the flow rate through the catheter 3, whether or not the flow rate through the liquid-sucking catheter is not more than the preset threshold of the suction stop flow rate is judged. In cases where the flow rate through the liquid-sucking catheter is judged to be not more than the preset threshold of the suction stop flow velocity, the control section 24 sets the integrated injection volume and the integrated suction volume to 0, and stops the driving of the liquid-sucking pump.

The following describes specific operations for a time chart illustrating a first control operation of the *in vivo* temperature control system for appropriately controlling the internal esophageal temperature while maintaining the amount of a liquid remaining in the esophagus constant during treatment of arrhythmia by a catheter ablation procedure.

### (Step 1: Process of Comparison of Temperature Information with Threshold)

The operator nasally or orally inserts the catheter 3 and the temperature probe 4 into the esophagus. After a catheter ablation procedure for ablation of the cardiac tissue begins in a position close to the left atrium, the internal esophageal temperature gradually increases in the vicinity thereof, and the internal esophageal temperature as measured by each temperature sensor 42 of the temperature probe 4 also gradually increases. In the control section 24, a threshold of the internal esophageal temperature at which the sending of the cooled liquid (cooling water) is begun can be preliminarily set by operating the touch-screen display 51 of the monitor 5, and the control section 24 constantly carries out the process of comparing the temperature information of the temperature sensor 42 with the threshold.

### (Step 2: Operation of Sending Liquid (Cooling Water))

When the temperature information from at least one of the pluralities of temperature sensors 42 has reached the threshold, the control section 24 outputs a driving command regarding the liquid-sending rate and the liquid-sending time to the pump 21. As a result, the liquid (cooling water) is sent from the liquid storage section 23 to the catheter 3 through the liquid-sending-sucking dual-purpose tube 6. Thus, when the liquid (cooling water) reaches the site at an increased temperature in the esophagus, the inside of the esophagus can be cooled by heat exchange with the liquid (cooling water). The amount of the liquid (cooling water) sent is controlled based on the liquid-sending rate and the liquid-sending time, which may be preset in the control section 24. The liquid (cooling water) is preferably sent in a short time. More specifically, the liquid is preferably sent at a liquid-sending rate of 1 mL/min to 300 mL/min.

### (Step 3: Operation of Keeping Liquid (Cooling Water) in Esophagus)

The operation time of Step 3 may be preset in the control section 24 such that Step 4 is begun after the set time has passed. In another method that may be employed, the internal esophageal temperature is monitored, and Step 4 is begun at the time when the internal esophageal temperature has reached a preset temperature.

### (Step 4: Calculation of Difference)

The control section 24 can determine, based on the driving history of the pump 21, and save the integrated amount of the volume of a liquid released to the outside of the catheter 3, in other words, the injection volume released to the inside of the esophagus, and the integrated amount of the volume of a liquid sucked to the inside of the catheter 3, in other words, the suction volume sucked from the inside of the esophagus, and calculates the difference (Δ) by subtracting the integrated suction volume from the integrated injection volume. By this, the amount of the liquid remaining in the esophagus can be estimated.

### (Step 5: Operation of Suction of Liquid Remaining in Esophagus)

In cases where the difference (Δ), in other words, the amount of the liquid remaining in the esophagus, exceeds the preset threshold, the control section 24 outputs a driving command to the pump 21 according to a preset suction flow rate, and starts suction of the liquid. After the start of suction, the control section 24 calculates the suction volume based on the driving history of the pump 21, and continues the driving of (suction by) the pump 21 until the difference (Δ) reaches a preset threshold. In suction of the liquid from the esophagus, since suction of the liquid in a short time may cause esophageal injury due to excessive suction, the liquid is preferably sucked at a lower rate. Specifically, the suction is preferably carried out at a suction rate of 1 mL/min to 100 mL/min.

In cases where during suction, a negative pressure which is an internal tube pressure of not more than a set threshold, is detected, in other words, where there is no more liquid that can be sucked from the esophagus, the esophagus is flattened and the catheter 3 is obstructed, and as a result, the internal pressure in the liquid-sending-sucking dual-purpose tube 6 is a negative pressure, the control section 24 sets the integrated injection volume and the integrated suction volume to 0, judging that the difference (Δ) is 0 and stops the driving of the pump 21. In cases where the liquid is sucked from the esophagus, a part of the liquid that has been injected may drain into the stomach positioned downstream, resulting in the possibility of occurrence of the gap between the calculated difference (Δ) and the amount of the liquid actually remaining in the esophagus. Thus, the driving of (suction by) the pump 21 can be stopped at the timing when no liquid remains in the esophagus. It is preferable that the control section 24 judges that the difference (Δ) is 0 when the internal pressure in the liquid-sending-sucking dual-purpose tube 6 is within the range of -10 kPaG to -90 kPaG.

In embodiments in which the *in vivo* temperature control device 2 comprises a flow rate meter 25 that can measure the flow rate of a liquid flowing inside the liquid-sending-sucking dual-purpose tube 6, the method of judging that there is no more liquid sucked during suction may be as follows. In cases where the flow rate of a liquid flowing in the liquid-sending-sucking dual-purpose tube 6 is measured by the flow rate meter 25, and the obtained flow rate reaches a preset threshold or less, in other words, in cases where there is no more liquid that can be sucked from the esophagus, and the flow rate of a liquid flowing inside the liquid-sending-sucking dual-purpose tube 6 decreases to the threshold or less, the control section 24 sets the integrated injection volume and the integrated suction volume to 0, judging that the difference (Δ) is 0, and stops the driving the pump 21.

In cases where the temperature information from at least one of the plurality of temperature sensors 42 reaches the threshold during suction, the control section 24 preferably outputs a driving command for liquid sending to the pump 21 and allows a liquid (cooling water) to be sent from the liquid storage section 23 to the catheter 3 through the liquid-sending-sucking dual-purpose tube 6, in order to prioritize cooling to prevent heating and damaging to the esophagus. In particular, in cases where the pump 21 plays both roles of liquid sending to the catheter 3 and of sucking, the control section 24 preferably switches the driving command to the pump 21 from suction to liquid sending.

Even if the difference (Δ) does not exceed a preset threshold, but in cases where the temperature detected by the temperature sensor 42 is the preset threshold or more, or where the time since injection exceeds a preset time, suction may be started. In this case, suction may be done until the difference (Δ) is a preset threshold or less, or a preset volume of a liquid may be sucked.

After the operation of Step 5, the process proceeds to Step 1 again, and then Steps 1 to 4 are repeated. By this, the esophageal temperature can be appropriately maintained, and the amount of the liquid remaining in the esophagus can be maintained constant. Since the first control operation is a control operation for cases where the internal esophageal temperature increases due to heat ablation of cardiac muscle by a radiofrequency ablation procedure or the like, the circuit is prepared such that the control section 24 judges reaching of the threshold when the esophageal temperature has exceeded the threshold. However, in cases where the internal esophageal temperature decreases due to a cryoablation procedure or the like, the internal esophageal temperature can be controlled by an operation which is the same as the first control operation described above except that heated water is used as the liquid to be sent, and that the circuit is prepared such that the control section 24 judges reaching of the threshold when the esophageal temperature has become lower than the threshold.

### INDUSTRIAL APPLICABILITY

The present invention can be applied in medical fields in which control of temperature rising or cooling in a living body is required, and is particularly applicable to the case where the temperature raised during catheter ablation procedure is cooled.

### DESCRIPTION OF SYMBOLS

1: *in vivo* temperature control system, 2: *in vivo* temperature control device, 3: catheter, 4: temperature probe, 5: monitor, 6: liquid-sending-sucking dual-purpose tube, 7: waste liquid section, 21: pump, 22: pressure sensor, 23: liquid storage section, 24: control section, 31: tube section, 32: valved connector, 41: shaft section, 42: temperature sensor, 43: handle section, 44: connection cable, 51: touch-screen display, 61: bulge section, 64: connection port, 221: contact-type displacement gauge, 231: Peltier device, 321: port, 322: valve, 431: connector, 621: three-way check valve, 631: needle

## Claims

1. An *in vivo* temperature control system, comprising:
a catheter insertable into a living body;
a temperature sensor that can measure the temperature in the living body;
a liquid storage section for storing a liquid;
a pump for supplying the liquid from the liquid storage section to the catheter; and
a control section for controlling the driving of the pump;
wherein the control section calculates the difference obtained by subtracting the integrated amount of the liquid sucked to the inside of the catheter from the integrated amount of the liquid released to the outside of the catheter, and allows the pump to operate and suck the liquid into the catheter when the difference exceeds a preset threshold, and then stop the driving of the pump when the difference reaches the preset threshold or less.

2. The *in vivo* temperature control system of claim 1, comprising:
a pressure sensor for detecting the internal pressure of the catheter,
wherein the control section, when sucking the liquid into the catheter, sets the integrated amount of the liquid released to the outside of the catheter and the integrated amount of the liquid sucked to the inside of the catheter to 0 when the pressure value obtained by the pressure sensor reaches a set threshold or less.

3. The *in vivo* temperature control system of claim 1, comprising:
a flow velocity meter or a flow rate meter that can measure the flow rate of the liquid flowing inside the catheter;
wherein the control section, when sucking liquid from the outside of the catheter, sets the integrated amount of the liquid released to the outside of the catheter and the integrated amount of the liquid sucked to the inside of the catheter to 0 when the flow velocity or flow rate value obtained by the flow velocity meter or the flow rate meter reaches a preset threshold or less.

4. The *in vivo* temperature control system of any one of claims 1 to 3,
wherein the control section selects whether the liquid in the liquid storage section is released or the liquid is sucked and drives the pump based on a signal detected by the temperature sensor or a preset time.
